# EUROPEAN PATENT APPLICATION

(11) **EP 4 393 528 A1**
(43) Date of publication of application: **03.07.2024**
(21) Application number: 22217236.3
(22) Date of filing: 29.12.2022
(51) Int. Cl.: A61M 1/16, A61J 1/10

(54) **FLEXIBLE BAG FOR DIALYSIS CONCENTRATES WITH SEALED OVERPOUCH**

(71) Applicant: Gambro Lundia AB, 226 43 Lund (SE)
(72) Inventor: Vartia, Christian, Antero, SE 24764 Veberöd (SE); Dioos, Bart, Michiel, 3050 Oud-Heverlee (BE); Dendal, Frédéric, Eugène, Louis, 1040 Etterbeek (BE); Lin, Rongsheng, Buffalo Grove, IL 60089 (US)
(74) Representative: PGA S.p.A., Milano, Succursale di Lugano

(57) **Abstract**

The present invention relates to a disposable flexible bag for dialysis concentrate comprising a primary bag and an overpouch designed to ensure that the overpouch remains on the primary bag during use of the concentrate to prevent water evaporation that could lead to an out-of-specification situation towards the end of the in-use period.

## Description

### FIELD OF THE INVENTION

The present invention relates to a disposable flexible bag for a dialysis concentrate comprising a primary bag and an overpouch. The bag arrangement is designed to ensure that the overpouch remains on the primary bag during use of the concentrate to prevent water evaporation that could lead to an out-of-specification situation towards the end of the in-use period. The invention targets foremost concentrates intended for multi-day use, where water loss could be problematic.

### STATE OF THE ART

Peritoneal dialysis ("PD") is one type of dialysis therapy commonly used to treat loss of kidney function. Peritoneal dialysis uses dialysis solutions (also called dialysis fluids) that are infused into a patient's peritoneal cavity through a catheter implanted in the cavity. The dialysis fluid contacts the patient's peritoneal membrane located in the peritoneal cavity. Waste, toxins and excess water pass from the patient's bloodstream through the peritoneal membrane and into the dialysis fluid. The transfer of waste, toxins, and water from the bloodstream into the dialysis fluid occurs due to diffusion and osmosis, i.e., an osmotic gradient occurs across the peritoneal membrane. Used dialysis fluid is drained from the patient's peritoneal cavity to remove waste, toxins and excess water from the patient. The above cycle is then repeated.

There are various types of peritoneal dialysis therapies, including continuous ambulatory peritoneal dialysis ("CAPD"), automated peritoneal dialysis ("APD") and continuous flow peritoneal dialysis ("CFPD").

CAPD is a manual dialysis treatment, in which the patient connects an implanted catheter to a drain and allows used dialysis fluid to drain from the peritoneal cavity. The patient then manually allows fresh dialysis fluid to flow from a solution bag, through the patient's indwelling catheter and into the patient's peritoneal cavity. The patient may then disconnect the catheter from the solution bag to allow the dialysis fluid to dwell within the peritoneal cavity to transfer waste, toxins and excess water from the patient's bloodstream to the dialysate solution. After a dwell period, the patient may repeat the above manual procedure. In CAPD the patient performs several drain, fill, and dwell cycles during the day, for example, about four times per day.

Automated peritoneal dialysis ("APD") is similar to CAPD in that the dialysis treatment includes a drain, fill, and dwell cycle. APD machines, however, automatically perform three to four cycles of peritoneal dialysis treatment, typically overnight while the patient sleeps. Typically, the APD machines are fluidly connected to an implanted catheter, to one or more solution bags and to a drain bag.

The APD machines pump fresh dialysate from the dialysate source, through the catheter, into the patient's peritoneal cavity and allow the dialysate to dwell within the cavity so that the transfer of waste, toxins and excess water from the patient's bloodstream to the dialysate solution can take place. The APD machines then pump spent dialysate from the peritoneal cavity, though the catheter, to the drain. APD machines are typically computer controlled so that the dialysis treatment occurs automatically when the patient is connected to the dialysis machine, for example, when the patient sleeps. That is, the APD systems automatically and sequentially pumps fluid into the peritoneal cavity, allows for a dwell, then pumps fluid out of the peritoneal cavity and repeats the procedure.

As with the manual process, several drain, fill, and dwell cycles occur during APD. A "last fill" is typically used at the end of APD, which remains in the peritoneal cavity of the patient when the patient disconnects from the dialysis machine for the day. APD frees the patient from having to manually perform the drain, dwell, and fill steps.

As discussed above, both CAPD and APD involve the use of solution bags that contain the fluid that will be pumped into the patient's peritoneal cavity. Preparing such bags requires a great deal of care and skill. The bags must not leak, the material from which they are made must be safe and sufficiently inert to avoid leaking substances into the fluid that could unintentionally be transferred to the patient, and the material must be strong and flexible enough to resist manufacture, handling and storage and should be opaque to allow visual inspection of the content, for example, in order to recognize unwanted discoloration or precipitation. Of course, bags must also be made within certain specifications. The solution bags as well as its content must also be sterilizable to a level such that the solution is safe to be delivered to the patient. Finally, the bags must also be labeled properly, so that the user or caregiver can be certain that the patient is receiving the correct PD solution.

The dialysis fluids used in all the above dialysis techniques contain mainly electrolytes like sodium, magnesium, calcium, potassium, an acid/base buffer system and optionally glucose or a glucose-like compound. All the components in dialysis fluids are selected to control the levels of electrolytes and the acid-base equilibrium within the blood and, in the case of glucose or glucose-like components, to remove waste materials from the blood by building up an osmotic gradient.

As is evident from the above, a patient needs high volumes of ready-to-use PD fluid each day for carrying out the necessary steps. These high volumes of the PD-fluid have to be prepared, shipped and then stored at the patient's home which adds cost to the treatment and adds burden for the patient and his or her caregivers. Therefore, ready-to-use PD fluids should preferably be prepared from different types of concentrates in the patient's home by mixing PD concentrates with purified water that is also prepared at the patient's home. In such case, the concentrates would typically be supplied in bags whereas the pure water needed for mixing is sourced from e.g. a water purification device that purifies the tap water. The concentrates are typically provided in two parts, as a buffer concentrate with electrolytes having a nominal dilution factor of about 20, and a glucose concentrate comprising 50% glucose, both having a volume of, for example, 1L. The electrolytes can also be provided separately from the buffer in two different concentrates.

Using multi-therapy concentrates enables cost savings due to shipping less products having lower weight and volume per treatment, and reducing waste due to less container material needed per treatment. It also lowers the patient burden since unpacking, placing and connecting the smaller and less heavy concentrate containers can be performed with larger intervals than daily changes, such as, for example 7-14 days.

In the medical field, primary bags are used to collect, store, transport, and ultimately deliver dialysis solutions. Oftentimes, these primary bags are placed into secondary containers such as an overpouch to maintain the integrity and volume of the agent contained within the primary container. The overpouch is usually removed or opened before use to uncover and connect the primary bag to a tubing set, as described, for example in WO2004/066857 and WO2006/116287 disclosing an easy-to-open overpouch enabling easy insertion and removal of the primary bag. The overpouch is then usually discarded.

As the mixing of such concentrates is predetermined, both in manual and automated procedure, based on the concentrate specifications, water evaporation from the bag must be avoided as long as the concentrates are used for preparing ready-to-use dialysis fluid for PD therapy.

During storage and transport of the concentrates, the water evaporation is essentially being prevented by the overpouch. The evaporation rate will however significantly increase as the overpouch is removed prior to and during use, which may take place at elevated temperatures or at least room temperatures and may last for up to four or five days. In addition, the evaporation rate in relation to the volume held by the concentrate bag will increase during the in-use time due to the increasing surface-to-volume ratio as the concentrate volume is decreasing faster than the container surface available for water diffusion out of the container. This means that the up-concentration rate of the concentrate will increase over the in-use time and be the highest towards the end of the in-use period. Especially towards the end of the in-use time there is thus a risk that the concentrate's concentration is out of specification.

Accordingly, there is a need for methods for reducing or preventing up-concentration of concentrates used for preparing PD fluids during in-use time, including providing disposable flexible bags for dialysis concentrates that are designed to prevent or at least to reduce water evaporation during the in-use time. The present invention is provided to solve this problem which may have severe consequences for the patient.

### SUMMARY OF THE INVENTION

The Applicant has faced the problem of developing a disposable flexible bag for dialysis concentrates that are suitable for preventing or at least reducing evaporation of water during the in-use time which carries the risk of up-concentration of the dialysis concentrate and, in consequence, preparation of dialysis fluid for use in PD that does not have the final concentration required for therapy.

After extensive experimentation, the Applicant has found a solution allowing to maintain the overpouch which can be used over the complete lifetime of the concentrate product, including sterilization, shipping, storage and use, including during the in-use time, thereby preventing or at least reducing water evaporation from and up-concentration of the concentrate during use in PD therapy.

The solution comprises a primary bag that holds the concentrate fluid, wherein the primary bag is contained in a sealed overpouch intended to protect the primary bag and its outlet connector from contamination and to prevent water loss during storage and use, while allowing easy and safe use of the concentrate bag with a PD cycler.

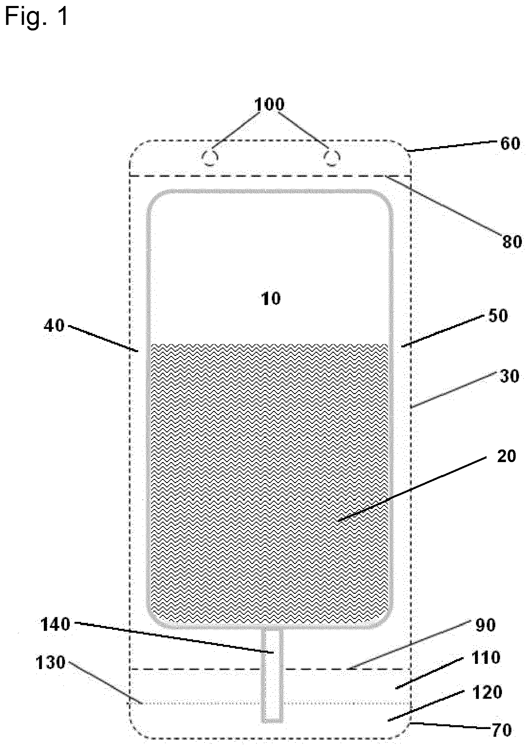

The overpouch is permanently sealed around and encloses the primary bag, wherein it includes two sealed side portions, a sealed upper portion and a sealed lower portion. The sealed lower portion of the overpouch comprises a fixed portion and a removable portion (Fig. 1). The sealed upper portion is closed with a permanent seal that is not opened during use of the concentrate and while the outlet connector is connected to the line leading to the PD cycler. The permeant seal spans the outlet connector in the upper portion thereof and is tightly fixed to the connector in order to close off the lower sealed portion of the overpouch from contact with ambient air.

The outlet connector of the primary bag accordingly extends through the sealed lower portion of the overpouch (Fig. 1) including extending through the fixed and removable portions, crossing the permanent seal of the lower sealed portion. The length of the outlet connector is thus partly comprised in the fixed portion of the sealed lower portion of the overpouch and partly comprised in the removable portion of the sealed lower portion of the overpouch. The terminal end and the tip of the outlet connector is comprised in the removable portion of the sealed lower portion of the overpouch for allowing using it during treatment.

Prior to use, the user removes the removable portion of the sealed lower portion of the overpouch by tearing the overpouch apart. The connector tip thus becomes accessible and free to be connected with the solution generation device, generally a PD cycler.

The overpouch is thus never removed from the primary bag, which remains to be permanently sealed within the overpouch by the two side portions, the sealed upper portion and the fixed portion of the sealed lower portion. Equally, the connector remains covered while its functionality is not being limited by the overpouch. Its upper portion is located within the overpouch, that is tightly sealed around the connector. The lower portion is fully accessible. The sealing of the overpouch at the bottom end where the connector is located and tight fitting around the primary bag or pouch further increases the ease of handling as the overpouch cannot unintentionally be removed, twisted or pushed upwards to expose the primary bag.

The Applicant has found that the above-described solution prevents or substantially reduces water evaporation both during storage and use of the disposable flexible bag for dialysis concentrate and is well suited to be safely applied during therapy.

### BRIEF DESCRIPTION OF THE FIGURES

**Figure 1** shows the configuration of the flexible bag for dialysis concentrate according to the present invention before use.
**Figure 2** shows the configuration of the flexible bag for dialysis concentrate according to the present invention during use.
**Figure 3** shows the Cartesian diagram of weight loss percentage versus time of three bags containing electrolyte concentrates as described in Example 2.
**Figure 4** shows the Cartesian diagram of weight loss percentage versus time of three bags containing glucose concentrates as described in Example 2.

### DETAILED DESCRIPTION OF THE INVENTION

While this invention can be implemented in many different forms, specific embodiments thereof are shown in the drawings and will be further described herein with the understanding that the present disclosure is an exemplification of the principles of the invention and is not intended to limit the invention to the specific embodiments illustrated.

According to the present invention, a disposable flexible bag for storing and providing a dialysis concentrate for therapy is provided, that is designed for preventing or at least reducing evaporation during the in-use time and which meets the requirements set forth above.

The flexible bag according to the present invention comprises a primary bag permanently sealed within an overpouch. The term "permanently sealed", as used in the present description and claims, means that the primary bag remains enclosed within the overpouch throughout its lifetime, i.e., from production, storage, use and disposal thereof.

The lower portion of the sealed overpouch comprises a removable portion which can be easily teared apart to uncover the terminal end and the tip of the outlet connector, and a fixed portion which remains completely sealed to prevent the primary bag to be exposed to air, thereby preventing or reducing water evaporation through the primary bag.

The sealed overpouch should be strong enough to remain intact and substantially airtight throughout typical sterilization, transport and storage processes, and for the subsequent 7-10 days of use after tearing off the removable portion.

Further, it is important that the sealing strength is permanent and remains in place from initial construction of the sealed package until the package is intended to be used. This is a special concern when the package is intended to be subjected to extreme temperature fluctuations or mechanical stress prior to opening, such as during sterilization, packaging and transport. It is well-known that PD concentrates require sterilization prior to use, and that the most common heat-sterilization technique involves the use of an autoclave process to destroy microorganisms at about 120°C for periods of 15 to 30 minutes. In this regard, the sealing strength must withstand the high temperatures associated with heat-sterilization.

According to the present invention, a specific embodiment is illustrated in the enclosed figure 1.

**Fig. 1** shows a primary bag 10 containing a PD concentrate 20 enclosed into a sealed overpouch 30 having opposed first and second side portions 40, 50 and opposed sealed upper and lower portions 60, 70.

The sealed overpouch 30 is made by providing a tube of polymeric material having the appropriate diameter and length and sealing together the opposed upper and lower portions 60, 70 after having inserted the primary bag 10.

Alternatively, the sealed overpouch 30 can also be made by providing two sheets of polymeric material having the appropriate width and length and sealing together the first and second side portions 40, 50 and the opposed upper and lower portions 60, 70 after having interposed the primary bag 10 between such sheets.

The dotted lines 80 and 90 denominate the permanently sealed upper and lower rims of the secondary bag or permanent overpouch that remains completely closed also during use of the bag once the removable portion 120 has been removed and the fixed portion 110 is open at the bottom to allow accessing the connector.

Advantageously, the sealed upper portion 60 comprises hanger holes 100 to hang the bag to an appropriate holder. **Fig. 1** shows two hanger holes 100, but the sealed upper portion 60 can also comprise just one hanger hole or more than two hanger holes.

The sealed lower portion 70 comprises a fixed portion 110 and a removable portion 120. The removable portion 120 can be torn off along the dotted line 130, whereby the connector becomes accessible.

The primary bag 10 comprises an outlet connector 140 which is fluidly connected to the interior of the primary bag and which passes through the permanent seal 90 and the lower portion 70 of the overpouch 30.

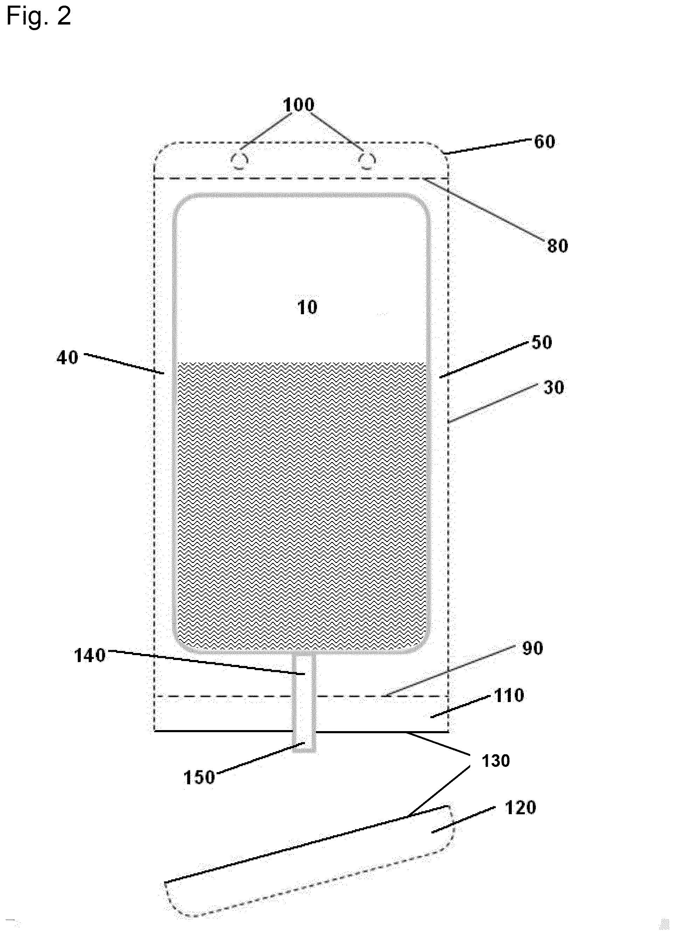

As illustrated in **Fig. 2****,** prior to use, the user removes the removable portion 120 of the sealed lower portion 70 by tearing off the removable portion 120 along the dotted line 130. The terminal portion and tip 150 of the outlet connector 140 thus becomes exposed and free to be connected to the solution generation device. A substantial portion of the outlet connector 140 remains covered by the fixed portion 110 of the lower portion 70 and within the permanently sealed overpouch that is closed with permanent seal 90.

The primary bag 10 and the overpouch 30 can be realized with a film made with any polymeric material conventionally employed for PD concentrate packages, such as polyvinyl chloride (PVC), polyolefins, such as for example polyethylene (PE) and polypropylene (PP), polyester, such as for example polyethylene terephthalate (PET), co-polyester, polyamide, co-polyamide, and polycarbonate. Polyolefin, polyester and polyvinyl chloride are the preferred polymeric materials. Preferably, the overpouch utilizes a material with low permeability for water vapor, such as polyethylene, polypropylene, or combinations of PE and PP, PVC/PVdC, or PVC/PCTFE laminations. The primary bag can preferably be made from PVC or cyclic olefin copolymers that offer an excellent barrier where halogen free packaging is preferred.

The films used for the primary and secondary bag, i.e., the overpouch, are preferably made from clear films to allow visual inspection of the contained concentrate. PE, for example, as well as PVC, are both clear materials that fulfill his requirement.

The film of polymeric material can have a monolayer structure or a multilayer structure comprising two or more layers of different composition. Useful examples of multilayered films are described, for example, in EP733472 A2 and EP738589 A2.

Product labels can be applied both to the primary and the secondary bag. In cases where the overpouch is made from a clear material, the application of the label to the primary bag can provide further advantages, since it is at lower risk to be physically damaged or rendered unreadable e.g., by moist. The product label can also be applied to the overpouch if such application is preferred, since the overpouch is permanently sealed together with the primary bag. In the latter case, the material used for the secondary bag or overpouch can also be chosen from opaque or untransparent materials such as high barrier foils made of multiple layers of different materials which are bonded together with adhesives or polyethylene and may include aluminum which provides for an especially low water vapor transmission rate (WVTR). The water vapor transmission rate is the measure of the passage of water vapor through a substance, such as the material used for the present primary or secondary bag. It is measured by grams per 100 inches square per 24 hours (g/100 inches²/24 hrs).

The present invention will be further illustrated below by means of several preparatory examples, which are provided for indicative purposes only and without any limitation of the present invention.

### EXPERIMENTAL PART

### Example 1

The sealed overpouch according to the present invention is able to substantially reduce the out-of-specification (OOS) risk throughout an in-use period of ten days for products having been stored for different periods of time (shelf lives) as illustrated in the following tables 1-3. The tables summarize the OOS probability for the concentrate's concentration of two major solutes (dextrose and sodium chloride) stored into a PVC 5L primary bag with and without overpouch. The overpouch was made from a polymeric material consisting of a blend of polyethylene and polypropylene.

**TABLE 1**

| 12 Month Shelf Life - 25°C | | | | |
|---|---|---|---|---|
| | **OOS Risk Without overpouch** | | **OOS Risk With overpouch** | |
| **Day** | **Dextrose** | **NaCl** | **Dextrose** | **NaCl** |
| 1 | 0.42% | 0.67% | 0.42% | 0.66% |
| 2 | 0.43% | 0.68% | 0.42% | 0.68% |
| 3 | 0.44% | 0.70% | 0.42% | 0.66% |
| 4 | 0.45% | 0.72% | 0.42% | 0.67% |
| 5 | 0.46% | 0.75% | 0.43% | 0.67% |
| 6 | 0.47% | 0.78% | 0.43% | 0.69% |
| 7 | 0.49% | 0.83% | 0.43% | 0.70% |
| 8 | 0.52% | 0.89% | 0.44% | 0.71% |
| 9 | 0.56% | 0.99% | 0.44% | 0.74% |
| 10 | 0.66% | 1.22% | 0.46% | 0.78% |

**TABLE 2**

| 18 Month Shelf Life - 25°C | | | | |
|---|---|---|---|---|
| | **OOS Risk Without overpouch** | | **OOS Risk With overpouch** | |
| **Day** | **Dextrose** | **NaCl** | **Dextrose** | **NaCl** |
| 1 | 0.68% | 1.27% | 0.67% | 1.25% |

| | **OOS Risk Without overpouch** | | **OOS Risk With overpouch** | |
|---|---|---|---|---|
| **Day** | **Dextrose** | **NaCl** | **Dextrose** | **NaCl** |
| 2 | 0.69% | 1.30% | 0.68% | 1.26% |
| 3 | 0.71% | 1.34% | 0.68% | 1.27% |
| 4 | 0.73% | 1.38% | 0.68% | 1.28% |
| 5 | 0.74% | 1.43% | 0.69% | 1.30% |
| 6 | 0.77% | 1.49% | 0.69% | 1.31% |
| 7 | 0.80% | 1.57% | 0.70% | 1.33% |
| 8 | 0.85% | 1.68% | 0.70% | 1.36% |
| 9 | 0.92% | 1.86% | 0.72% | 1.40% |
| 10 | 1.08% | 2.27% | 0.74% | 1.49% |

**TABLE 3**

| 24 Month Shelf Life - 25°C | | | | |
|---|---|---|---|---|
| | **OOS Risk Without overpouch** | | **OOS Risk With overpouch** | |
| **Day** | **Dextrose** | **NaCl** | **Dextrose** | **NaCl** |
| 1 | 1.11% | 2.35% | 1.10% | 2.32% |
| 2 | 1.13% | 2.41% | 1.10% | 2.34% |
| 3 | 1.15% | 2.46% | 1.10% | 2.35% |
| 4 | 1.18% | 2.53% | 1.11% | 2.37% |
| 5 | 1.21% | 2.62% | 1.12% | 2.39% |
| 6 | 1.25% | 2.85% | 1.12% | 2.42% |
| 7 | 1.30% | 3.03% | 1.13% | 2.46% |
| 8 | 1.37% | 3.33% | 1.14% | 2.50% |
| 9 | 1.48% | 3.33% | 1.16% | 2.57% |
| 10 | 1.72% | 3.99% | 1.20% | 2.72% |

The data of tables 1 to 3 clearly show that the out-of-specification (OOS) risk has been significantly reduced when using the overpouch of the present invention.

### Example 2

Another test was made with a set of three PVC 1-liter electrolyte concentrate bags and a set of three PVC 1-liter glucose concentrate bags with and without overpouch. The overpouch was made from a polymeric material consisting of a blend of polyethylene and polypropylene.

The test was conducted as follows. At first, the original weight of each bag was recorded. For each set, one bag was kept with the closed overpouch (bag A), one bag was kept with the opened overpouch (bag B), by removing the removable portion 120 of the sealed lower portion 70 and exposing the outlet connector, and one bag was kept without overpouch (bag C). Each bag was hung in free air and was weighed with a few days apart during a total test period of 35 days.

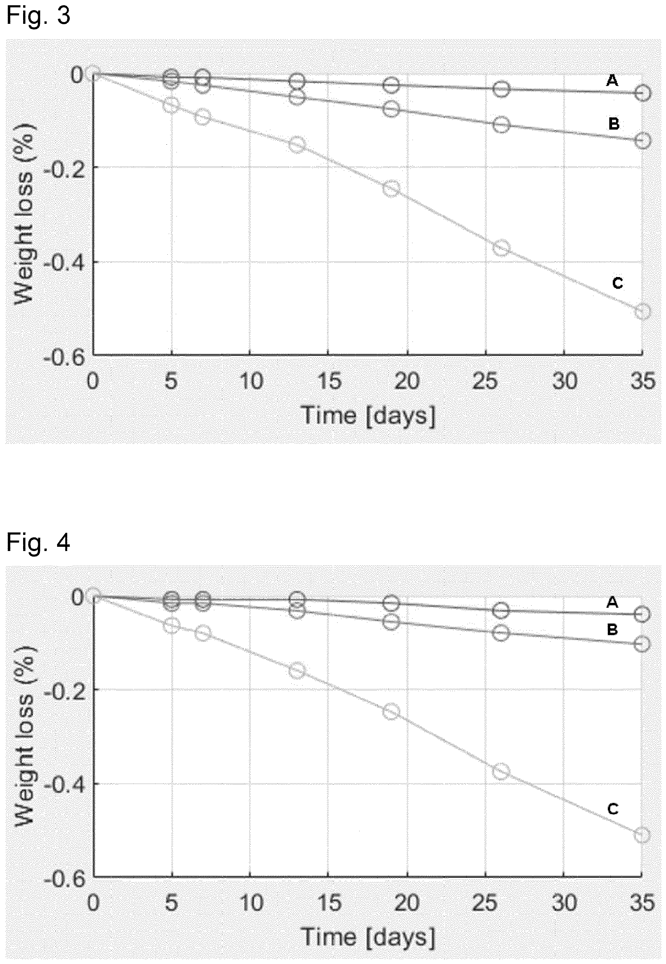

The results in percent weight loss are summarized in **Figure 3** for the set of electrolyte concentrate bags and in **Figure 4** for the set of glucose concentrate bags, where lines A, B and C represent the trend lines of bags A, B and C, respectively.

The results demonstrate that the evaporation rate is strongly reduced in bags A and B, maintaining the overpouch in the closed or opened in-use configuration according to the invention, compared to bags C without the overpouch. The calculated evaporation rate reduction was 71% for the electrolyte concentrate and 79% for the glucose concentrate.

It is understood that, given the above description of the embodiments of the invention, various modifications may be made by one skilled in the art. Such modifications are intended to be encompassed by the claims below.

## Claims

1. A disposable flexible bag for dialysis concentrates comprising a primary bag (10) with an outlet connector (140) and an overpouch (30), **characterized in that** said primary bag (10) is permanently sealed within said overpouch (30).

2. The disposable flexible bag for dialysis concentrate according to claim 1, **characterized in that** said overpouch comprises a sealed lower portion (70) comprising a fixed portion (110) and a removable portion (120).

3. The disposable flexible bag for dialysis concentrate according to claim 2, **characterized in that** said outlet connector (140) is enclosed within said sealed lower portion (70) during storage.

4. The disposable flexible bag for dialysis concentrate according to claim 2, **characterized in that** said outlet connector (140) is exposed when removing said removable portion (120).

5. The disposable flexible bag for dialysis concentrate according to claim 1, **characterized in that** said overpouch comprises a sealed upper portion (60).

6. The disposable flexible bag for dialysis concentrate according to claim 5, **characterized in that** said sealed upper portion (60) comprises one or more hanger holes (100), preferably two hanger holes (100).

7. The disposable flexible bag for dialysis concentrate according to claim 1, **characterized in that** said primary bag (10) contains a PD concentrate (20).

8. The disposable flexible bag for dialysis concentrate according to claim 1, **characterized in that** said overpouch (30) has opposed first and second sealed side portions (40, 50).

9. The disposable flexible bag for dialysis concentrate according to any one of preceding claims 1 to 8, **characterized in that** said primary bag (10) and said overpouch (30) are made with a polymeric material selected from the group consisting of polyvinyl chloride (PVC), polyolefins, such as for example polyethylene (PE) and polypropylene (PP), polyester, such as for example polyethylene terephthalate (PET), co-polyester, polyamide, co-polyamide, and polycarbonate.

10. The disposable flexible bag for dialysis concentrate according to claim 9, **characterized in that** said primary bag (10) is made with PVC.

11. The disposable flexible bag for dialysis concentrate according to claim 9, **characterized in that** said overpouch (30) is made with a polymeric material selected from the group consisting of polyethylene (PE), polypropylene (PP), and a mixture thereof.
